# EUROPEAN PATENT SPECIFICATION

(11) **EP 3 965 735 B1**
(45) Date of publication and mention of the grant of the patent: **30.08.2023**
(21) Application number: 20720419.9
(22) Date of filing: 17.04.2020
(51) Int. Cl.: A61K 9/08, A61K 47/02, A61K 31/4439, A61P 1/00, A61P 1/04, A61P 25/20

(54) **PHARMACEUTICAL FORMULATION COMPRISING A BENZIMIDAZOLE**
PHARMAZEUTISCHE ZUBEREITUNG ENTHALTEND EIN BENZIMIDAZOL
COMPOSITION PHARMEUTIQUE COMPRENANT UN BENZIMIDAZOLE

(30) Priority: 08.05.2019 GB 201906473
(43) Date of publication of application: 16.03.2022
(73) Proprietor: Alkaloid AD Skopje, 1000 Skopje (MK)
(72) Inventor: KRSTESKA, Ljiljana, 1000 Skopje (MK); KAZANDZIEVSKA, Elena, 1250 Debar (MK); WILLIS, Andrew, Usk NP15 1AB (GB)
(74) Representative: CSY London
(86) International application number: PCT/EP2020/060869
(87) International publication number: WO 2020/224936

(56) References cited:
- WO-A1-01/51050
- WO-A2-03/061584
- US-A1- 2005 054 682
- US-A1- 2011 015 233

## Description

### Technical Field

The present invention is directed to a two-part pharmaceutical formulation comprising a substituted benzimidazole, more specifically Omeprazole. The present invention also relates to preparations of such pharmaceutical formulations.

### Background of the Invention

Proton pump inhibitors are a class of medicaments whose main action is a pronounced and long-lasting reduction of stomach acid production. They have the general structure (I)

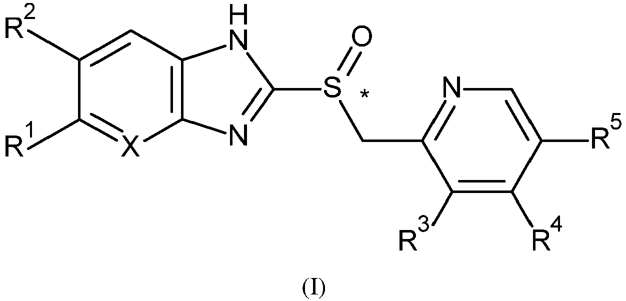

| **Name** | **R¹** | **R²** | **R*³*** | **R⁴** | **R⁵** | **X** |
|---|---|---|---|---|---|---|
| Timoprazole | H | H | H | H | H | CH |
| Omeprazole | OCH₃ | H | CH₃ | OCH₃ | CH₃ | CH |
| Lansoprazole | H | H | CH₃ | OCH₂CF₃ | H | CH |
| Pantoprazole | CF₂HO | H | OCH₃ | OCH₃ | H | CH |
| Rabeprazole | H | H | CH₃ | O(CH₂)₃OCH₃ | H | CH |
| Tenatoprazole | OCH₃ | H | CH₃ | OCH₃ | CH₃ | N |
| Picoprazole | CH₃ | CO₂CH₃ | CH₃ | H | H | CH |

Additionally, certain of the compounds shown in the table are used as single enantiomers (at the chiral centre indicated * in formula (I)). Esomeprazole is a single enantiomer of omeprazole. Dexlansoprazole is a single enantiomer of lansoprazole.

Omeprazole is a substituted benzimidazole, 6-methoxy-2- [[(4-methoxy-3, 5-dimethyl-2-pyridinyl) methyl] sulfinyl]-1H-benzimidazole that reduces gastric acid production by irreversibly inhibiting the H⁺/K⁺ ATPase (proton pump) at the secretory surface of the gastric parietal cells. The formula of omeprazole is

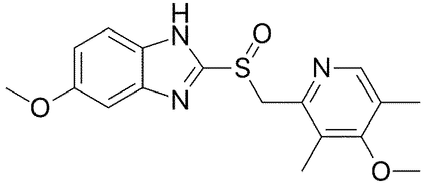

The proton pump is directly responsible for secreting H⁺ ions into the lumen of the stomach. By inhibiting the proton pump, omeprazole regulates the final step of hydrogen ion production in the pathway for gastric acid secretion in the gastrointestinal tracts of mammals.

Omeprazole and other proton pump inhibitors (PPIs) are used in the treatment of a number of conditions which require reduction in acid production by proton pump inhibition, such as gastritis, gastroesophageal reflux disease (GERD), dyspepsia, peptic ulcer disease, laryngopharyngeal reflux, gastric and duodenum ulceration and Zollinger-Ellison syndrome.

Typically, omeprazole is formulated as tablets or capsules for oral administration. However, such formulations present problems for patients who may be unable and/or unwilling to swallow capsules or tablets. This form is also undesirable for paediatric use.

A liquid formulation of omeprazole for oral administration has proven to be problematic due to the unstable nature of the active ingredient. Omeprazole is an acid labile compound and therefore, rapidly degrades in acidic conditions such as the environment found in the stomach. Omeprazole is known to degrade with a half-life of less than 10 minutes in an environment having a pH of below 4.0; at pH 6.5, the half-life is 18 hours and at pH 11, about 300 days.

An alternative to the above formulations is the provision of omeprazole as a solid suspension, in which granules of omeprazole are mixed with a suitable carrier. The suspension needs to be prepared immediately before use and it needs a strict control in the way it is prepared. As a result, there is a chance that the medicine may be rendered ineffective if not prepared correctly. Some patients also find the suspension very unpleasant to consume. Since the shelf-life is very short for omeprazole formulated as a suspension, it is also difficult to batch manufacture these.

One solution is to provide a liquid suspension of omeprazole which does not require controlled preparation. However, such suspensions are required to be of high viscosity to ensure that the microgranules of omeprazole are fully suspended in solution. This high viscosity makes it difficult to measure the correct dosage. Also, such formulations provide slow release of omeprazole and hence, they are limited in use.

It is therefore desirable to provide a stable pharmaceutical formulation comprising omeprazole or other PPIs which also addresses the problems identified above.

The present invention is related to a pharmaceutical formulation comprising omeprazole, esomeprazole, or other proton pump inhibitors, or a mixture thereof, which provides cost effective means for the treatment of the aforementioned conditions and is convenient to prepare. It also provides alternatives to patients unable and/or unwilling to ingest capsules. The present formulation is stable, easily dissolvable and it allows immediate release and rapid absorption of the therapeutic agent without it being degraded by the stomach acid.

### Summary of the Invention

According to a first aspect, the invention provides a pharmaceutical formulation comprising:
a first composition being a liquid having a pH of from10 to 12 comprising a substituted benzimidazole at a concentration between 0.5mg/ml to 5mg/ ml, a buffering agent, a base and optionally an antioxidant; and
a second composition being a liquid having a pH of from 7 to 9 comprising a diluent and a pH modifying agent;
whereby the first and the second compositions are configured to be combined immediately before use to provide a combined liquid medicament of pH 8 to 9.

According to a second aspect of the invention, there is provided a pharmaceutical formulation comprising:
a first composition at a pH of from 10 to12 comprising a therapeutic agent at a concentration of from 0.5mg/ml to 5mg/ml, a buffer and optionally an antioxidant; and
a second composition of pH 7-9 comprising a diluent and a pH modifying agent;
whereby the first and the second compositions are configured to be combined immediately before use to achieve a combined solution of pH 8-9.

According to another aspect, the pharmaceutical formulation of the present invention is provided for use as a medicament to treat a condition selected from the group consisting of gastritis, gastroesophageal reflux disease, dyspepsia, peptic ulcer disease, laryngopharyngeal reflux, gastric and duodenum ulceration or Zollinger-Ellison syndrome.

According to another aspect, there is provided a kit comprising
a first compartment containing a first composition as defined herein;
a second compartment containing a second composition as defined herein; wherein the first and second containers are adapted to allow fluid communication between them on actuation to form a combined liquid medicament as defined in any one of claims 1 to 15.

### Detailed Description of the Invention

In accordance with the present invention, there is provided a two-part pharmaceutical formulation comprising a first and a second composition wherein the first composition comprises a therapeutic agent, an antioxidant and a buffer, and the second composition comprises a diluent and a pH modifying agent, and wherein the two compositions are combined to achieve a combined liquid medicament for immediate oral use.

In a preferred embodiment, the therapeutic agent is selected from the group of proton pump inhibitors. The proton pump inhibitor is preferably one of those defined in Formula (I) above, including pharmaceutically acceptable salts, esters and enantiomeric mixtures thereof. More preferably the therapeutic agent is selected from the group consisting of omeprazole, esomeprazole, lansoprazole, dexlansoprazole, timoprazole, picoprazole, pantoprazole, rabeprazole, and tenatoprazole. These proton pump inhibitors may be used singularly or in combination with each other. These inhibitors provide lasting reduction of gastric acid production by blocking the hydrogen/potassium adenosine triphosphate enzyme system (H⁺/K⁺ ATPase) of the gastric parietal cells.

Preferably, the therapeutic agent is selected from omeprazole, esomeprazole, pantoprazole and pharmaceutically acceptable salts, enantiomers, alkaline salts, enantiomers, hydrates, or derivatives thereof. In a preferred embodiment, the therapeutic agent is omeprazole or a pharmaceutically acceptable salt. A pharmaceutically acceptable salt of the therapeutic agent may comprise alkali metal salts such as sodium, lithium and potassium salts. A pharmaceutically acceptable salt of the therapeutic agent may also comprise alkaline earth and transition metal salts such as calcium and magnesium salts.

The concentration of the therapeutic agent present in the first composition is between about 0.5mg/ml to about 5mg/ml. Preferably, the amount of the therapeutic agent in the first composition may be between about 2 mg/ml to about 4 mg/ml. More preferably, the therapeutic agent is at a concentration of about 2mg/ml. This therapeutic agent is found to be stable at about pH 8 to 14, particularly at about pH 10 to 13. More particularly, the therapeutic agent is stable at a pH of from 11.5 to 12.5. In a more preferred embodiment, the therapeutic agent is present at about pH 12.

A feature of the invention is that the first composition comprises a buffering agent and a base. It will be apparent to the skilled person that a buffering agent is normally capable of maintaining the pH of a solution within a given range (the buffering range) within which addition of strong base does not alter the pH greatly. However, the first composition comprises sufficient base to exceed the buffering capacity, and as a consequence, is rather basic.

The therapeutic agent may be present in the first composition in any suitable form, such as in the form of a suspension, liquid, solution, powder, granules, dry powder, dry granules, or microgranules. The therapeutic agent may additionally be provided with a coating, such as, for example, an enteric coating which would be stable from degradation when in contact with an acidic medium, such as present in the stomach, but would break down rapidly at a higher pH, when in the gastrointestinal tract for absorption. In a preferred embodiment, the therapeutic agent is provided in the form of a liquid, preferably a solution. Preferably, the liquid is an aqueous liquid.

The therapeutic agent may be in the form of powder, granules or microgranules suspended in an appropriate suspension medium, or dissolved in an appropriate solvent. In a preferred embodiment, the medium is aqueous.

Suitable antioxidants for use in the first composition are selected from ascorbic acid derivatives, thiol derivatives, sulphites, sodium sulfates, synthetic hindered phenols like propyl gallate, butylated hydroxyanisole (BHA), butylated hydroxytoluene (BHT), natural hindered phenols like tocopherols, or a mixture thereof. In a more preferred embodiment, the antioxidant is a thiol derivative, more preferably, N-acetyl-L-cysteine. The antioxidant lends stability to the therapeutic agent and prevents acid degradation. The amount of antioxidant in the composition may be from about 0.05% (w/v) to about 0.5% (w/v), preferably from about 0.1% (w/v) to about 0.3% (w/v) of the first composition.

The second composition of the present invention also comprises a diluent and a pH modifying agent. The diluent may be any suitable liquid diluent, for instance, water, a polar organic solvent, an aqueous solvent, or a mixture thereof. Preferably, the diluent is water. The amount of the diluent depends on the final volume of the formulation required depending on the concentration of the therapeutic agent.

In the context of this invention, the pH modifying agent serves to regulate the pH of the formulation. Thus, the pH modifying agent is capable of modifying the pH of the final formulation to obtain a desired pH that prevents the therapeutic agent form being degraded by the acidic gastric fluids, and is suitable for oral administration. In a preferred embodiment, the pH of the final formulation (combined solution) is between 6 and 10. In a more preferred embodiment, the pH of the final formulation is between 8 and 9. In a more preferred embodiment, the pH of the combined solution is between pH 8.5 and 9. In a more preferred embodiment, the pH of the combined solution is about 9. The pH modifying agent may be selected from alkali metal or alkaline earth metal carbonates or bicarbonates, alkali metal or alkaline earth metal hydroxides, alkali metal or alkaline earth metal oxides, alkali metal or alkaline earth metal phosphates, citrates, acetates and a mixture thereof. Preferably, the pH-modifying agent is sodium bicarbonate.

The pH modifying agent ensures that when the first and second compositions are mixed, the buffering agent is brought back within the buffering range, and hence the pH of the combined liquid medicament is consistent and stable on administration.

In another embodiment, the first and second compositions may comprise pharmaceutically acceptable excipients, additives or carriers. For the purpose of this invention, the excipients may be one or more of dispersing agents, viscosity building agents, sequestering agents, flavouring agents, sweeteners and preservatives.

Preservatives (or stabilising agents) preserve the active pharmaceutical ingredient by sequestering it and are referred to as secondary preservative agents for the purpose of the present invention. The preservatives may be selected from EDTA or a salt thereof; sugar alcohol such as glycerol and sorbitol; polyoxyethylene 20 sorbitan monooleate such as polysorbate 80, polyoxyethylene 20 sorbitan monolaurate like polysorbate 20; disodium phosphate; or a mixture thereof. Preferably, the stabilising agent is sodium EDTA or disodium phosphate. More preferably, the stabilising agent is sodium EDTA. The stabilising agent may be present in amout from about 0.01 % (w/v) to about 0.5% (w/v), more preferably, about 0.1% (w/v).

Viscosity building agents (or thickening agents) serve to regulate the viscosity of the formulation and decrease the bitterness of the active pharmaceutical ingredient. The amount of the thickening agents depends on the viscosity of the final formulation and/or the therapeutic agent required. DV2T RV Viscometer is used for apparent viscosity measurement in the present invention. In a preferred embodiment, the viscosity of the final pharmaceutical formulation (combined solution) of the present invention may be from about 200 centipose to about 600 centipose. In a preferred embodiment, the viscosity of the final formulation is from about 300 centipose to about 400 centipose. The viscosity of the first composition may be from about 150 centipose to about 450 centipose or from about 200 centipose to about 300 centipose. The viscosity of the second composition may be from about 350 centipose to about 650 centipose, or from about 400 centipose to about 550 centipose. The thickening agent may be selected from carageenans, powdered cellulose, methylcellulose, hydroxyl ethyl cellulose, hydroxyl propyl cellulose, carboxymethylcellulose, sodium carboxymethylcellulose / microcrystalline cellulose mixtures, ion-exchange cross-linked polyacrylic polymers, polysaccharides, starches, carbomers, or a mixture thereof. In a preferred embodiment, the thickening agent is a combination of polysaccharide and sodium carboxymethylcellulose. Xanthan gum is a preferred polysaccharide.

The therapeutic agent used in this invention is preferably sparingly soluble in water at pH 7. The purpose of the surfactants, solvents and co-solvents in this invention is to improve the solubility of the active pharmaceutical ingredients. In one embodiment, the surfactants may be selected from polyol esters, sorbitan derivatives, polyoxyethylene esters, poloxamers, lauryl sulfates, dodecyl sulfates, quaternary ammonium compounds or a mixture thereof. The surfactants may be about 0.1% (w/v) to about 1.0% (w/v), more preferably, the surfactants may be about 0.5% (w/v).

In another embodiment, the solvents and co-solvents may be selected from glycerol, sorbitol, propylene glycol, alcohol, polyethylene glycols, or a mixture thereof. In a preferred embodiment, the solvents and co-solvents may be sugar alcohol. The solvents and co-solvents may be present in amount from about 5% (w/v) to about 20% (w/v), more preferably, from about 10% (w/v) to about 15% (w/v).

The anti-foaming agent prevents the formation of any bubbles or foaming. Suitable antifoaming agents may be selected from silicone based antifoaming agent such as, simethicone or its emulsion or suspension, and non-silicone anti-foaming agents like polypropylene based polyether dispersions, castor oil, fatty alcohol esters, glycerides and a mixture thereof. The anti-foaming agent may be present in an amount from about 0.01 % (w/v) to about 0.08% (w/v).

In another embodiment, the diluent may also comprise osmotic agents, preservatives, dispersing agents, sweeteners, and flavouring agents.

The purpose of the osmotic agents used in this invention is to give the combined formulation an osmolality in the range of from about 1000 to about 3000 mOsmol /kg. In a preferred embodiment, the osmolality may be from about 1000 to about 1500 mOsmol /kg or from about 1500 to about 2500 mOsmol /kg or from about 1500 to about 2200 mOsmol/kg. The presence of osmotic agents within this range ensures that the pharmaceutical formulation achieved has acceptable hypertonicity such that the formulation can be consumed in an undiluted form. The osmotic agents may be selected from dextrose, mannitol, sorbitol, glycerol, sodium chloride and sodium sulfate or a combination thereof.

The pharmaceutical formulation of this invention may comprise preservatives which ensure that the formulation is protected from microbial contaminants. The preservatives may be selected from parabens, benzoic acid and salts thereof, sorbic acid and salts thereof, sodium sulfate, potassium sulfate, propylene glycol, EDTA and salts thereof. Preferably, the preservative is sodium methylparaben or domiphen bromide. The amount of the preservative may be about 0.1% (w/v) to about 0.2% (w/v), more preferably, about 0.15% (w/v).

The function of the dispersing agents is to aid sparingly soluble components to dissolve properly. Suitable dispersing agents for use in this invention are glycerol, sorbitol, propylene glycol, polyethylene glycols, alcohol and combinations thereof.

The sweetening agents and flavouring agents are used to mask the bitter taste of the active pharmaceutical ingredient and thus, a formulation with good palatability and acceptability is provided. Suitable sweetening agents for use in the present invention are sugars, sugar alcohols, sodium saccharide, aspartame, acesulfame potassium, stevia, sodium cyclamate, liquorice extract, glycyrrhizin, maple extract and locust bean extract. Suitable flavouring agents may be selected from taste-mask sweetness flavour, sucralose, menthol, lemon, orange, peach, cinnamon, black current, cherry and chocolate. The sweetening agents and flavouring agents may be present in an amount from about 1.0% (w/v) to about 0.01 % (w/v). More preferably, these agents may be present in an amount from about 0.25% (w/v), 0.2% (w/v), 0.15% (w/v), or 0.015% (w/v).

Preferably, the first and the second composition are both provided in the form of a solution. In a more preferred embodiment, the combined liquid medicament is homogeneous.

The first composition and second composition are provided in volumes such that when combined, the volume of combined liquid medicament is acceptable for a single oral dose. Typically, the volume of combined liquid medicament is between 1 and 50 ml, preferably 5 to 30 ml, more preferably 10 to 25 ml, such as around 15 ml. Accordingly, the volumes of the first and second compositions are selected to provide such a total volume of combined liquid medicament.

In another embodiment the present invention provides a kit that comprises the first and second compositions, such that both the compositions are stored separately and are combined to form a combined liquid immediately prior to use. The resulting solution preferably has a pH of from 8 to 9 and this pH stability is maintained for at least 2 hours.

The first and second compositions, kept separately, can be stored for at least 12 months and the active pharmaceutical agent maintains its activity for at least 12 months.

The kit may comprise a removable barrier between the two compositions. The removable barrier is configured to prevent contact between the two compositions whereby the removal of the removable barrier allows combining of the two compositions immediately before consumption to provide the combined solution. In a preferred embodiment, the barrier is a breakable membrane.

Optionally, the removable barrier may be reusable as long as it can separate the two compositions and be removed so as to allow mixing of the two compositions. For example, the removable barrier may be two separate containers for the two compositions having removable openings such as caps or lids. Alternatively, the removable barrier may have a feature of a single container comprising the two compositions separated by the removable barrier. Preferably, the removable barrier is made of plastic or glass. More preferably, the removable barrier is made of amber high-density polyethylene.

The pharmaceutical formulation of the present invention is provided for use in the treatment of a number of conditions such as gastritis, gastroesophageal reflux disease, dyspepsia, peptic ulcer disease, laryngopharyngeal reflux, gastric and duodenum ulceration, and Zollinger-Ellison syndrome whereby the condition is treated by proton pump inhibition. The pharmaceutical formulation of the present invention is suitable for adults and more suitable for children.

In use, the clinician or patient admixes the first and second compositions to form a combined liquid, which is then directly orally administered to the patient.

The formulations and kits of the invention combine a long shelf-life with minimal degradation of the active substance, together with a convenient and palatable dosage form for administration.

Following examples further describe the invention:

### Example 1.

**Table 1**

| **Omeprazole 2mg/1ml** - **First composition** | |
|---|---|
| **INGREDIENTS** | **% w/v** |
| Omeprazole | 0.200 |
| Sugar alcohol | 10.00 |
| Polysaccharide | 0.30 |
| Polyoxyethylene 20 sorbitan monooleate | 0.50 |
| EDTA Na | 0.10 |
| Thiol derivative | 0.30 |
| NaH₂PO₄ | 0.180 |
| Simethicone emulsion | 0.08 |
| Sodium Hydroxide 10 % solution | qs pH 11.5-12.5 |
| Water purified | ad 100.0 ml |

**Table 2**

| **Second composition** | |
|---|---|
| **INGREDIENTS** | **% w/v** |
| Sodium carboxymethylcellulose | 0.80 |
| Sugar alcohol | 15.0 |
| Polysaccharide | 0.30 |
| Sodium bicarbonate | 4.20 |
| Sucralose | 0.35 |
| Sodium methylparaben | 0.15 |
| Flavor Mint | 0.20 |
| Flavor Lemon | 0.25 |
| Taste-mask sweetness flavor | 0.15 |
| Water, purified | ad 100.00 ml |

In an alternative, the second composition is identical, but in place of sodium methylparaben instead comprises domiphen bromide 0.025%w/v, and 0.3%w/v flavor mint.

In Example 1, the first composition comprises 2mg/ml of omeprazole, sodium dihydrogen phosphate as a buffer, sodium hydroxide as a base and a thiol derivative as an antioxidant. The second composition comprises water as diluent and sodium bicarbonate as a pH modifying agent.

### Example 2.

**Table 3**

| **Omeprazole 2mg/1ml- First composition** | |
|---|---|
| **INGREDIENTS** | **% w/v** |
| Omeprazole | 0.200 |
| Sugar alcohol | 10.00 |
| Polysaccharide | 0.30 |
| Polyoxyethylene 20 sorbitan monooleate | 0.50 |
| EDTA Na | 0.10 |
| Thiol derivative | 0.30 |
| NaH₂PO₄ | 0.180 |
| Simethicone Emulsion | 0.08 |
| Sodium Hydroxide 10 % solution | qs pH 11.5-12.5 |
| Water purified | ad 100.0 ml |

**Table 4**

| **Second composition** | |
|---|---|
| **INGREDIENTS** | **% w/v** |
| Sodium carboxymethylcellulose | 0.80 |
| Sugar alcohol | 15.0 |
| Polysaccharide | 0.30 |
| Sodium bicarbonate | 4.20 |
| Sucralose | 0.35 |
| Sodium methylparaben | 0.15 |
| Flavor Orange flexarome | 0.20 |
| Flavor Cinnamon | 0.015 |
| Flavor Lemon | 0.20 |
| Taste-mask sweetness flavor | 0.15 |
| Water, purified | ad 100.00 ml |

In an alternative, the second composition is identical, but in place of sodium methylparaben instead comprises domiphen bromide 0.025%w/v, and instead of 0.2%w/v, 0.3%w/v flavor mint.

In Example 2, the first composition comprises 2mg/ml of Omeprazole, sodium dihydrogen phosphate as a buffer, sodium hydroxide as a base and a thiol derivative as an antioxidant. The second composition comprises water as diluent and sodium bicarbonate as a pH modifying agent.

### Example 3.

**Table 5**

| **Omeprazole 2mg/1ml - First composition** | |
|---|---|
| **INGREDIENTS** | **% w/v** |
| Omeprazole | 0.20 |
| Polyol liquid | 30.00 |
| Sugar Alcohol | 20.00 |
| Polysaccharide | 0.30 |
| Polyoxyethylene 20 sorbitan monooleate | 0.50 |
| EDTA Na | 0.10 |
| Thiol derivative | 0.18 |
| KOH 10% solution | q.s.11 .5- 12.5 |
| Water, purified | ad 100.00 ml |

**Table 6**

| **Second composition** | |
|---|---|
| **INGREDIENTS** | **% w/v** |
| Maize Starch | 2.00 |
| Sugar alcohol | 15.00 |
| Xanthan gum - Polysaccharide | 0.30 |
| Sodium bicarbonate | 4.20 |
| Sucralose | 0.15 |
| Sodium methylparaben | 0.15 |
| Flavor strawberry | 0.25 |
| Water, purified | ad 100.00 ml |

In an alternative, the second composition is identical, but in place of sodium methylparaben instead comprises domiphen bromide 0.025%w/v, and instead of 0.2%w/v, 0.3%w/v flavor mint.

In Example 3, the first composition comprises 2mg/ml of Omeprazole, potassium hydroxide buffer and a thiol derivative as an antioxidant. The second composition comprises water as diluent and sodium bicarbonate as a pH modifying agent.

### Example 4.

The first composition comprising omeprazole as mentioned in the above examples is kept at around pH 12 in separation from the second composition. The stability data of the omeprazole of the first composition as provided in Example 1 is shown below:

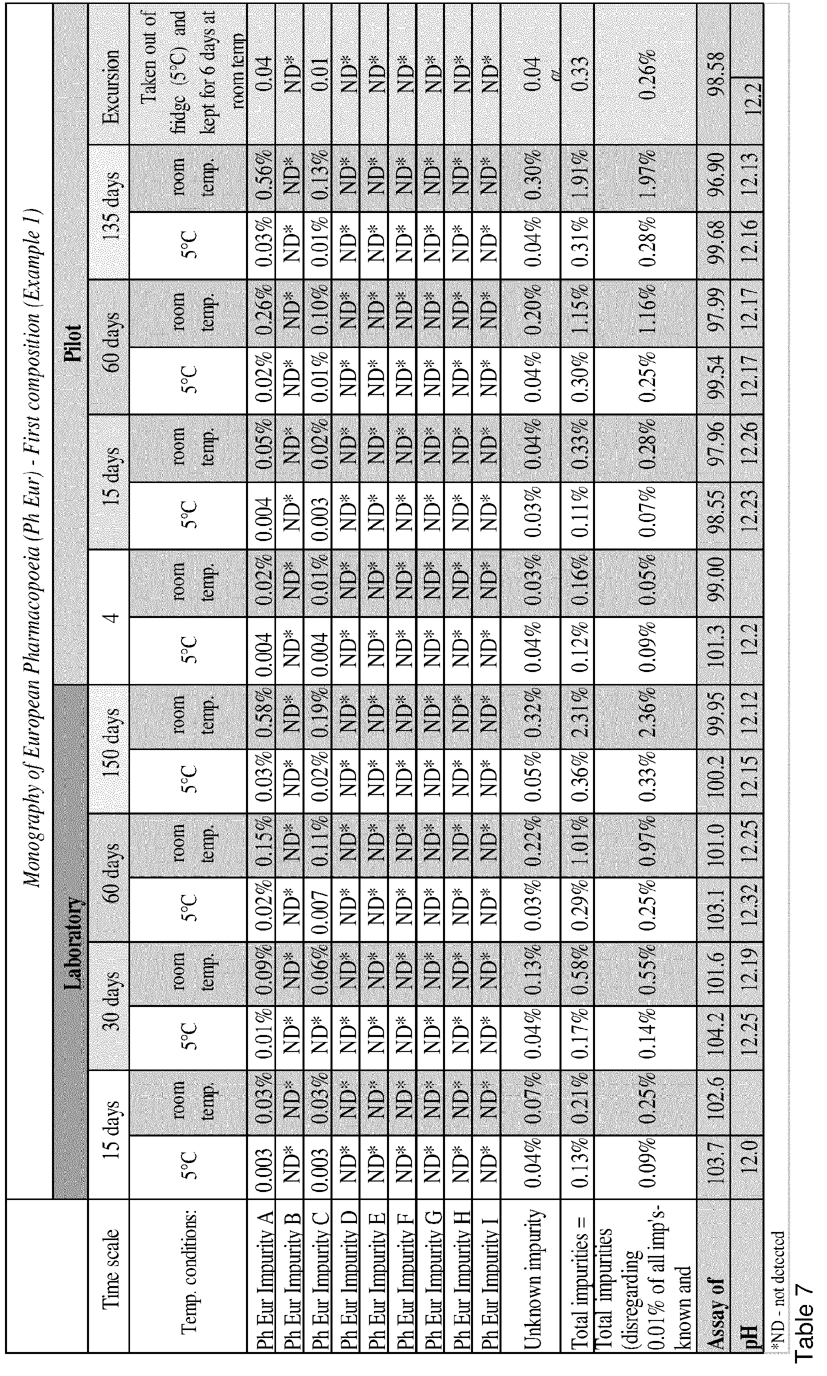

### Example 5.

When the first and second composition of the present invention are combined immediately before use, a combined solution is achieved which has a pH of around 8-9. Below is the pH stability data of the combined solution during the period of 0 min, 15 min, 60 min and 120 min immediately after mixing the first and the second composition.

**Table 8**

| | Supporting pH data of combined solution of Example 1 | | | |
|---|---|---|---|---|
| | Initial - 0min | after 15min | after 60min | after 120min |
| sample 1 | 8.88 | 8.87 | 8.89 | 8.89 |
| sample 2 | 8.82 | 8.83 | 8.84 | 8.84 |
| sample 3 | 8.86 | 8.86 | 8.87 | 8.88 |
| sample 4 | 8.86 | 8.87 | 8.87 | 8.89 |
| sample 5 | 8.87 | 8.87 | 8.88 | 8.90 |

From the results presented in the table above, it is shown that the pH value of the combined solution is stable for at least 120 min.

### Example 6

Formulation of Omeprazole 20mg/15 ml oral solution comprises omeprazole as active substance as formulation appears as off white to pale yellow solution with mint and lemon odour.

### Composition of the Drug Product

Omeprazole 20 mg/ 15 ml oral solution comprises of Omeprazole 4 mg/ml solution (first composition) and a diluent (second composition). The components of the omeprazole 4mg/ml solution (5 ml) and the diluent (10 ml) is presented in Table 9 and Table 10.

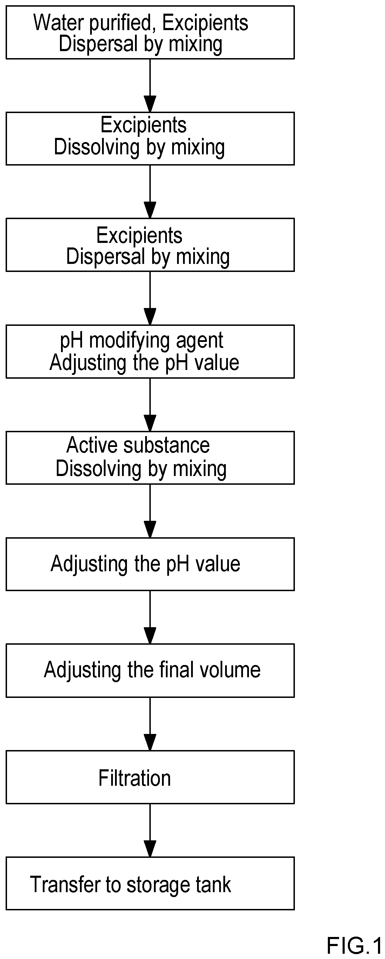

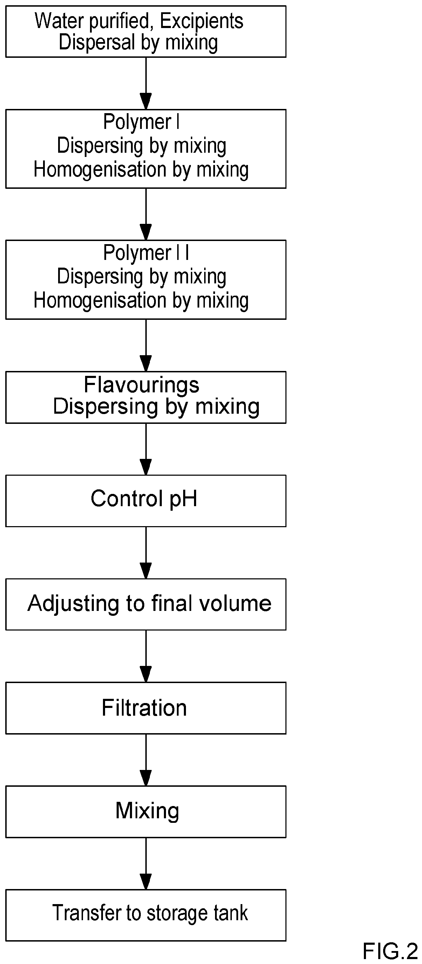

The manufacturing process of the first composition is shown in Figure 1. The manufacturing process of the second composition is shown in Figure 2.

**Table 9: Components of Omeprazole 4 mg/ ml oral solution (first composition)**

| **Omeprazole 4mg/ml solution (5 ml)** | | | | |
|---|---|---|---|---|
| **Name of Component** | **Amount mg/ml** | **Amount g/100 ml** | **Function** | **Quality reference¹** |
| Omeprazole | 4.00 mg | 0.400g | Active substance | Ph.Eur. |

| **Excipients:** | | | | |
|---|---|---|---|---|
| Glycerol | 100.00 mg | 10.00 g | dispersing agent | Ph.Eur. |
| Xanthan gum | 3.00 mg | 0.30 g | viscosity building agent | Ph.Eur. |
| Disodium edetate | 1.00 mg | 0.10 g | sequestering agent, preservative synergistic agent | Ph.Eur. |
| N - Acetyl L - cysteine | 2.20 mg | 0.30 g | antioxidant, stabilisation agent | Ph.Eur. |
| Emulsion Simethicone 30% | 0.80 mg | 0.08 g | antifoaming agent | In - house |
| Sodium Dihydrogen Phosphate | 2.60 mg | 0.18 g | buffering agent | Ph.Eur. |
| Sodium Hydroxide as 10% solution | 1.20 mg | q.s. 11.5-12.5 | buffering agent | Ph.Eur. |
| Water, purified | ad 1.00 ml | ad 100.00 ml | solvent | Ph.Eur. |

| | | | | |
|---|---|---|---|---|
| ¹ References: Current edition of the European Pharmacopoeia ; In -house Specification | | | | |

**Table 10: Components of Diluent 10ml (second composition)**

| **Diluent (10ml)** | | | | |
|---|---|---|---|---|
| **Name of component** | **Amount mg/ml** | **Amount g/100 m**l | **Function** | **Quanlity reference¹** |
| **Excipients:** | | | | |
| Glycerol | 150.00 mg | 15.00 g | Diluent dispersing agent, sweetener | Ph.Eur. |
| Xanthan gum | 4.00 mg | 0.30 g | viscosity building agent | Ph.Eur. |
| Sodium carboxymethyl-cellulose | 80.00 mg | 0.80 g | viscosity building agent | Ph.Eur. |
| Sucralose | 3.50 mg | 0.35 g | sweetening agent | Ph.Eur. |
| Sodium Bicarbonate | 42.0 mg | 4.20 g | pH modifying agent | Ph.Eur. |
| Sodium methyl para-hydroxybezote | 1.50 mg | 0.15 g | preservative | Ph.Eur. |
| Lemon flavour | 2.50 mg | 0.25 g | flavouring agent | In - house |
| *Ingredients:* | | | | |
| Maize maltodextrin | | | | |
| Flavouring components | | | | |
| Menthol flavour | 2.00 mg | 0.20 g | flavouring agent | In - house |
| *Ingredients:* | | | | |
| Acacia gum (gum Arabic) E 414 | | | | |
| Flavouring components | | | | |
| Taste masking flavour | 1.50 mg | 0.15 g | flavouring agent | In - house |
| *Ingredients:* | | | | |
| Water | | | | |
| Propylene glycol E 1520 | | | | |
| Flavouring components | | | | |
| Water, purified | ad 1.00 ml | ad 100.00 ml | solvent | Ph.Eur. |

| | | | | |
|---|---|---|---|---|
| ¹ References: current edition of the European Pharmacopoeia ; IN House Specification In an alternative, the second composition is identical, but in place of sodium methylparaben instead comprises domiphen bromide (0.25mg/ml; 0.025 g/100ml), and instead of menthol flavor, flavour mint (3.00mg/ml; 0.3g/100ml). | | | | |

### Example 7

Formulation of Pantoprazole 10mg/15 ml oral solution comprises pantoprazole as active substance, as Pantoprazole sodium sesquihydate.

The formulation appears as off white to pale yellow solution with mint and lemon odour.

Pantoprazole 10 mg/ 15 ml oral solution comprises of Pantoprazole 2 mg/ml solution (first composition) and diluent (second composition). The composition of the pantoprazole 2 mg/ml solution (5 ml) is presented in Table 11.

**Table 11: Composition of Pantoprazole 2 mg/ ml solution (first composition)**

| **Pantoprazole 2mg/ml solution (5ml)** | | | | |
|---|---|---|---|---|
| **Name of component** | **Amount mg/ml** | **Amount g/100 ml** | **Function** | **Quality reference¹** |
| Pantoprazole (as a sodium sesquihyrate ) | 2.000 mg | 0.200 g | active substance | Ph.Eur. |
| | 2.260 mg | 0.226 g | | |

| **Excipients:** | | | | |
|---|---|---|---|---|
| Glycerol | 100.00 mg | 10.00 g | dispersing agent | Ph.Eur. |
| Xanthan gum | 3.00 mg | 0.30 g | viscosity building agent | Ph.Eur. |
| Disodium edetate | 1.00 mg | 0.10 g | sequestering agent, preservative synergistic agent | Ph.Eur. |
| N - Acetyl L - cysteine | 2.20 mg | 0.30 g | antioxidant, stabilisation agent | Ph.Eur. |
| Sodium Dihydrogen Phosphate | 2.60 mg | 0.18 g | buffering agent | Ph.Eur. |
| Sodium Hydroxide as 10% solution | 1.20 mg | q.s. 11.5-12.5 | buffering agent | Ph.Eur. |
| Water, purified | ad 1.00 ml | ad 100.00 ml | solvent | Ph.Eur. |

| | | | | |
|---|---|---|---|---|
| Composition of Diluent 10ml (second composition) - as Example 3, Table 6. ¹ References: Current edition of the European Pharmacopoeia ; In -house Specification | | | | |

### Example 8

Formulation of Pantoprazole 20 mg/15 ml oral solution comprises pantoprazole as the active substance, as Pantoprazole sodium sesquihydate.

The formulation appears as off white to pale yellow solution with mint and lemon odour.

Pantoprazole 20 mg/ 15 ml oral solution comprises of Pantoprazole 4 mg/ml solution (first composition) and diluent (second composition). The composition of the pantoprazole 4 mg/ml solution (5 ml) is presented in Table 12.

**Table 12: Composition of Pantoprazole 4 mg/ ml solution (first composition)**

| Pantoprazole 4mg/ml solution (5ml) | | | | |
|---|---|---|---|---|
| Name of component | Amount mg/ml | Amount g/100ml | Function | Quality reference¹ |
| Pantoprazole (as a sodium sesquihyrate ) | 4.000 mg | 0.400 g | active substance | Ph.Eur. |
| | 4.520 mg | 0.452 g | | |

| Excipients: | | | | |
|---|---|---|---|---|
| Glycerol | 100.00 mg | 10.00 g | dispersing agent | Ph.Eur. |
| Xanthan gum | 3.00 mg | 0.30 g | viscosity building agent | Ph.Eur. |
| EDTA Na | 1.00 mg | 0.10 g | sequestering agent, preservative synergistic agent | Ph.Eur. |
| N - Acetyl L - cysteine | 2.20 mg | 0.30 g | antioxidant, stabilisation agent | Ph.Eur. |
| Sodium Dihydrogen Phosphate | 2.60 mg | 0.18 g | buffering agent | Ph.Eur. |
| Sodium Hydroxide as 10% solution | 1.20 mg | q.s. 11.5-12.5 | buffering agent | Ph.Eur. |
| Water, purified | ad 1.00 ml | ad 100.00 ml | solvent | Ph.Eur. |

| | | | | |
|---|---|---|---|---|
| Composition of Diluent 10ml (second composition) - as Example 3, Table 6.. ¹ References: Current edition of the European Pharmacopoeia ; In -house Specification | | | | |

### Example 9

Formulation of Esomeprazole 10mg/15ml oral solution comprising active substance esomeprazole as magnesium trihydrate. It appears as pale yellow solution with mint and lemon odour.

Esomeprazole 10 mg/ 15 ml oral solution consists of esomeprazole (as magnesium trihydrate) 2 mg/ml solution (first composition) and diluent (second composition). The components of the esomeprazole 2 mg/ml solution (5 ml) are presented in Table 13.

**Table 13: Composition of Esomeprazole 2 mg/ ml solution (first composition)**

| **Esomeprazole 2mg/ml solution (5 ml)** | | | | |
|---|---|---|---|---|
| **Name of constituents** | **Amount mg/ml** | **Amount g/100ml** | **Function** | **Quality reference¹** |
| Esomeprazole (as magnesium trihydrate) | 2.000 mg | 0.200 g | active substance | Ph.Eur. |
| | 2.223 mg | 0.223 g | | |

| **Excipients:** | | | | |
|---|---|---|---|---|
| Glycerol | 100.00 mg | 10.00 g | dispersing agent | Ph.Eur. |
| Xanthan gum | 3.00 mg | 0.30 g | viscosity building agent | Ph.Eur. |
| Disodium edetate | 1.00 mg | 0.10 g | sequestering agent, preservative synergistic agent | Ph.Eur. |
| N - Acetyl L - cysteine | 2.20 mg | 0.30 g | antioxidant, stabilisation agent | Ph.Eur. |
| Sodium Dihydrogen Phosphate | 2.60 mg | 0.18 g | buffering agent | Ph.Eur. |
| Sodium Hydroxide as 10% solution | 1.20 mg | q.s. 11.5-12.5 | buffering agent | Ph.Eur. |
| Water, purified | ad 1.00 ml | ad 100.00 ml | solvent | Ph.Eur. |

| | | | | |
|---|---|---|---|---|
| Composition of Diluent 10ml (second composition) - as Example 3, Table 6.. ¹ References: Current edition of the European Pharmacopoeia ; In -house Specification | | | | |

### Example 10

Formulation of Esomeprazole 20mg/15 ml oral solution comprises active substance esomeprazole as magnesium tryhidrate. The formulation appears as pale yellow to yellow solution with mint and lemon odour.

Esomeprazole 20 mg/ 15 ml comprises Esomeprazole 4 mg/ml solution (first composition) and diluent (second composition). Composition of the esomeprazole 4 mg/ml solution (5 ml) are presented in Table 14.

**Table 14: Composition of Esomeprazole 4 mg/ ml solution (first composition)**

| **Esomeprazole 4mg/ ml solution (5ml)** | | | | |
|---|---|---|---|---|
| **Name of contituents** | **Amount mg/ml** | **Amount g/100 ml** | **Function** | **Quality reference¹** |
| Esomeprazole (as magnesium tryhidrate) | 4.00 mg | 0.400 g | active substance | Ph.Eur. |
| | 4.46 mg | 0.446 g | | |

| **Excipients:** | | | | |
|---|---|---|---|---|
| Glycerol | 100.00 mg | 10.00 g | dispersing agent | Ph.Eur. |
| Xanthan gum | 3.00 mg | 0.30 g | viscosity building agent | Ph.Eur. |
| Disodium edetate | 1.00 mg | 0.10 g | sequestering agent, preservative synergistic agent | Ph.Eur. |
| N - Acetyl L - cysteine | 2.20 mg | 0.30 g | antioxidant, stabilisation agent | Ph.Eur. |
| Emulsion Simethicone 30% | 0.80 mg | 0.08 g | antifoaming agent | In - house |
| Sodium Dihydrogen Phosphate | 2.60 mg | 0.18 g | buffering agent | Ph.Eur. |
| Sodium Hydroxide as 10% solution | 1.20 mg | q.s. 11.5-12.5 | buffering agent | Ph.Eur. |
| Water, purified | ad 1.00 ml | ad 100.00 ml | solvent | Ph.Eur. |

| | | | | |
|---|---|---|---|---|
| ¹ References: Current edition of the European Pharmacopoeia ; In -house Specification Composition of Diluent 10ml (second composition) - as Example 6. | | | | |

### Example 11

Omeprazole 20mg/15ml (Example 6), Pantoprazole 1 0mg/15 ml (Example 7), Pantoprazole 20mg/15 ml (Example 8), Esomeprazole 10mg/15 ml (Example 9) or Esomeprazole 20mg/15ml (Example 10) oral solution were prepared as single dose, ready to use oral liquid pharmaceutical products.

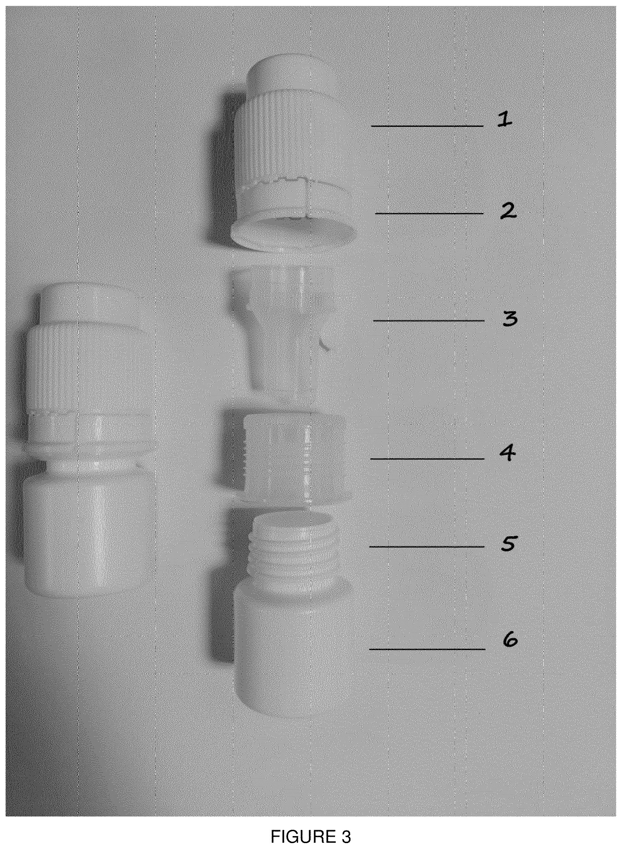

For the above mentioned products a dual - chamber single dose pack was used. This is shown in Figure 3. The first chamber is filled with the first composition 5 ml. The second chamber is filled with diluent (second composition) 10 ml, which are stored separately and mixed at time of administration. With reference to Figure 3, dual - chamber single dose pack comprises:

### Cap with tamper evident tear band

Cap (1) over the first chamber comprising a means to exert pressure onto the plunger (3) so as to partially rupture the breakable polymeric membrane (4) of the plug and deliver the solution into the container (6) with the second liquid composition, diluent for the Omeprazole solution in amount of 10ml.

### Tamper evident tear band (2)

The tamper evident tear band (2) ensures the overall integrity of the product until the time of the administration.

A first chamber composed comprising:

### Plunger - first container (3)

Plunger (3) adapted to fit into a plug (4) having a top flat surface, containing a first composition Omeprazole oral solution in amount of 5 ml.

### Plug (4)

The plug (4) with breakable polymeric membrane, adapted to fit into the opening bottle liner (5) from the lower end into a cap (1) from the upper end. Second chamber comprising:

### Opening bottle liner (5)

### Container - second container (6)

A second chamber in the form of a container (6) provided with an opening bottle liner (5) at an upper end, comprising liquid diluent for the Omeprazole oral solution in amount of 10 ml. In container (6) the two liquid compositions are mixed at the time of the administration.

### Example 12

### Measurement of Viscosity of First and Second Composition and Combined Solution

The method for viscosity measurement of above mentioned solutions is described below:
**Apparatus:** Rotating viscometer (Brookfield DV2T LV Viscometer) with spindle SC4-18
**Procedure:** Place 6.7ml of the solution in the SC4-18 chamber. Immerse the SC4-18 spindle into the test solution and set the speed at **6 rpm.** After 1 minute had passed read out the value for viscosity. The solution was maintained at temperature of 23±3ºC. The measured value for viscosity of the solution must meet acceptance criteria 150- 450 mPa.s (cPs).

**Table 15. Results from viscosity of Pantoprazole 2mg/ml solution (Example 7 - first composition); spindle CS4 - 18**

| Speed (rpm) | Viscosity (cPs) | Torque (%) | Shear Stress Dyn/cm² | Shear Rate 1/s | Temperature °C |
|---|---|---|---|---|---|
| 6 | 281.94 | 56.4 | 22.33 | 7.92 | 23.8 |

**Table 16. Results from viscosity of Pantoprazole 4mg/ml solution (Example 8 - first composition); spindle CS4 - 18**

| Speed (rpm) | Viscosity (cPs) | Torque (%) | Shear Stress Dyn/cm² | Shear Rate 1/s | Temperature °C |
|---|---|---|---|---|---|
| **6** | 273.44 | 54.7 | 21.66 | 7.92 | 23.5 |

**Table 17. Results from viscosity of Omeprazole 4mg/ml solution (Example 6 - first composition); spindle CS4 - 18**

| Speed (rpm) | Viscosity (cPs) | Torque (%) | Shear Stress Dyn/cm² | Shear Rate 1/s | Temperature °C |
|---|---|---|---|---|---|
| 6 | 260.94 | 52.2 | 20.67 | 7.920 | 23.3 |

**Table 18. Results from viscosity of Esomeprazole 2mg/ml solution; spindle CS4 -18 (Example 9 - first composition)**

| Speed (rpm) | Viscosity (cPs) | Torque (%) | Shear Stress Dyn/cm² | Shear Rate 1/s | Temperature °C |
|---|---|---|---|---|---|
| **6** | 248.45 | 49.7 | 19.68 | 7.920 | 23.3 |

**Table 19. Results from viscosity of Esomeprazole 4mg/ml solution; spindle CS4 -18 (Example 10 - first composition)**

| Speed (rpm) | Viscosity (cPs) | Torque (%) | Shear Stress Dyn/cm² | Shear Rate 1/s | Temperature °C |
|---|---|---|---|---|---|
| **6** | 286.44 | 57.3 | 22.69 | 7.920 | 23.4 |

### Diluent 10 ml (second composition)

**Chosen method for viscosity measurement (upon the previous method optimisation) is displayed below:**
The method for viscosity measurement of diluent 10 ml of Examples 6-10 is described below:
**Apparatus:** Rotating viscometer (Brookfield DV2T LV Viscometer) with spindle SC4-18
**Procedure:** Place 6.7ml of the solution in the SC4-18 chamber. Immerse the SC4-18 spindle into the test solution and set the speed at **4 rpm.** After 1 minute has passed read out the value for viscosity. The solution was measured at temperature of 23±3ºC. The measured value for viscosity of the solution must meet acceptance criteria 350-650.

**Table 20. Results from viscosity of Diluent (second composition) 10ml, spindle CS4 -18**

| Speed (rpm) | Viscosity (cPs) | Torque (%) | Shear Stress Dyn/cm² | Shear Rate 1/s | Temperature °C |
|---|---|---|---|---|---|
| 4 | 394.69 | 49.7 | 15.72 | 5.28 | 23.1 |

The method for viscosity measurement of the combined solutions of Examples 6-8 are described below:
**Apparatus:** Rotating viscometer (Brookfield DV2T LV Viscometer) with spindle SC4-18.
**Procedure:** Place 6.7ml of the solution in the SC4-18 chamber. Immerse the SC4-18 spindle into the test solution and set the speed at **6 rpm.** After 1 minutes have passed read out the value for viscosity. The measurements were taken at temperature of 23±3ºC. The measured value for viscosity of the solution must meet acceptance criteria 300- 600 mPa.s (cPs).

**Table 21. Results from viscosity of Pantoprazole 10mg/15ml (Example 7) oral combined solution, spindle CS4 -18**

| Speed (rpm) | Viscosity (cPs) | Torque (%) | Shear Stress Dyn/cm² | Shear Rate 1/s | Temperature °C |
|---|---|---|---|---|---|
| 6 | 397.42 | 6.00 | 79.5 | 7.92 | 23.8 |

**Table 22. Results from viscosity of Pantoprazole 20mg/15ml (Example 8) oral combined solution, spindle CS4 - 18**

| Speed (rpm) | Viscosity (cPs) | Torque (%) | Shear Stress Dyn/cm² | Shear Rate 1/s | Temperature °C |
|---|---|---|---|---|---|
| 6 | 402.41 | 6.00 | 31.87 | 7.92 | 23.4 |

**Table 23. Results from viscosity of Omeprazole 20mg/15ml (Example 6) oral combined solution, spindle CS4 -18**

| Spear (rpm) | Viscosity (cPs) | Torque (%) | Shear Stress Dyn/cm² | Shear Rate 1/s | Temperature °C |
|---|---|---|---|---|---|
| 6 | 408.91 | 81.8 | 32.39 | 7.92 | 123.3 |

**Table 24. Results from viscosity of Esomeprazole (Example 9) 10mg/15ml oral combined solution, spindle CS4 -18**

| Speed (rpm) | Viscosity (cPs) | Torque (%) | Shear Stress Dyn/cm² | Shear Rate 1/s | Temperature °C |
|---|---|---|---|---|---|
| **6** | 325.93 | 65.2 | 25.81 | 7.920 | 23.4 |

**Table 25. Results from viscosity of Esomeprazole (Example 10) 20mg/15ml oral combined solution, spindle CS4 - 18**

| Speed (rpm) | Viscosity (cPs) | Torque (%) | Shear Stress Dyn/cm² | Shear Rate 1/s | Temperature °C |
|---|---|---|---|---|---|
| **6** | 343.43 | 68.7 | 27.20 | 7.920 | 23.4 |

### Example 13

### Measurement of Osmolality

Unlike solid pharmaceutical forms (capsules, tablets) that are taken with a certain amount of water (app.250 mL water), oral solutions are usually taken in an undiluted form. Oral liquid drugs are in principle hypertonic and they are taken without dilution. There are oral liquid pharmaceutical forms of drugs that have a very high osmolality value of 3500, 5000 or more mOsm/kg. If such liquid drugs are taken in an undiluted form, there is a potential risk of an osmotic diarrhoea, which is a pronounced in paediatric patient group.

For these reasons, the osmolality of omeprazole solutions were determined to confirm acceptable hypertonicity in order to ensure that the drug can be taken in an undiluted form.

### • Results:

Osmolality was measured with Osmometer Vogel 802. Measurements were performed on diluted samples in ratio of 1:10.

Obtained results for osmolality for Pantoprazole 2mg/ml solution, Pantoprazole 4mg/ml and Omeprazole 4mg/ml solution are presented below:

**Table 26.Results of osmolality of Pantoprazole 2mg/ ml solution (Example 7 - first composition)**

| | |
|---|---|
| **Parameter** | **Osmolality** |
| ***Limits*** | ***100-1500 mOsm*/*kg*** |
| **Temperature** | **25°C** |
| Measurement | **Results** |
| 1 | 1230 |
| 2 | 1220 |
| 3 | 1230 |

**Table 27. Results of osmolality of Pantoprazole 4mg/ ml solution (Example 8 - first composition)**

| | |
|---|---|
| **Parameter** | **Osmolality** |
| ***Limits*** | ***1000-1500 mOsm*/*kg*** |
| **Temperature** | **25°C** |
| Measurement | **Results** |
| 1 | 1810 |
| 2 | 1850 |
| 3 | 1840 |

**Table 28.Results of osmolality of Pantoprazole 4mg/ ml solution (Example 8 - first composition)**

| | |
|---|---|
| **Parameter** | **Osmolality** |
| ***Limits*** | ***1000-1500 mOsm*/*kg*** |
| **Temperature** | **25 °C** |
| Measurement | **Results** |
| 1 | 1100 |
| 2 | 1110 |
| 3 | 1110 |

The preferred range of osmolality for Pantoprazole 2mg/ml;4mg/ml and Omeprazole 4mg/ml solution is 1000 - 1500 mOsm/kg

**Table 29.Results of osmolality of Esomeprazole 2mg/ ml solution (Example 9 - first composition)**

| **Esomeprazole 2mg/ml solution** | |
|---|---|
| Parameter | Osmolality |
| *Limits* | *1000-1500 mOsm*/*kg* |
| Temperature | 25 °C |
| Measurement | Results |
| 1 | 1010 |
| 2 | 1090 |
| 3 | 1100 |

The preferred range of osmolality for Esomeprazole 2mg/ml solution; 4mg/ml and Esomeprazole 4mg/ml solution is 1000 - 1500 mOsm/kg.

**Table 30. Results of osmolality of Esomeprazole 4mg/ ml solution (Example 10 - first composition)**

| **Esomeprazole 2mg/ml solution** | |
|---|---|
| Parameter | Osmolality |
| *Limits* | *1000-1500 mOsm*/*kg* |
| Temperature | 25 ° |
| Measurement | Results |
| 1 | '1100 |
| 2 | 1160 |
| 3 | 1170 |

**Table 31. Results of osmolality of Diluent (second solution) 10ml**

| | |
|---|---|
| parameter | osmolality |
| *Limits* | *1500-2500 mOsm*/*kg* |
| Temperature | 25 °C |
| Measurement | Results |
| 1 | 1850 |
| 2 | 1880 |
| 3 | 1730 |

Preferred range of osmolality for diluent (second composition) is 1500 - 2500 mOsm/kg.

Obtained results for osmolality for Pantoprazole10mg/15ml, pantoprazole 20mg/15ml and omeprazole 20mg/15ml, esomeprazole 10mg/15 ml and esomeprazole 20mg/15 ml oral are presented below:

**Table 32.Results of osmolality of Omeprazole 20mg/15 ml (Example 6) combined oral solution.**

| **Omeprazole 2mg/ml oral solution** | |
|---|---|
| **Parameter** | **Osmolality** |
| ***Limits*** | ***1500-2500 mOsm*/*kg*** |
| **Temperature** | **25 °C** |
| **Measurement** | **Results** |
| 1 | 1100 |
| 2 | 1190 |
| 3 | 1180 |

**Table 33. Results of osmolality of Pantoprazole 10mg/15 ml (Example 7) combined oral solution**

| **Panoprazole 2mg/ml oral solution** | |
|---|---|
| **Parameter** | **Osmolality** |
| ***Limits*** | ***1500-2500 mOsm*/*kg*** |
| **Temperature** | **25 °C** |
| **Measurement** | **Results** |
| 1 | 1810 |
| 2 | 1850 |
| 3 | 1840 |

**Table 34. Results of osmolality of Pantoprazole 20mg/15 ml (Example 8) oral solution**

| **Pantoprazole 2mg/15ml oral solution** | |
|---|---|
| **Parameter** | **Osmolality** |
| ***Limits*** | ***1500-2500 mOsm*/*kg*** |
| **Temperture** | **25 °C** |
| **Measurement** | **Results** |
| 1 | 1850 |
| 2 | 1870 |
| 3 | 1860 |

**Table 35. Results of osmolality of Esomeprazole 10mg/ 15ml (Example 9) solution**

| **Esomeprazole 10mg/15ml solution** | |
|---|---|
| Parameter | Osmolality |
| *Limits* | *1500-2500 mOsm*/*kg* |
| Temperature | 25 °C |
| Measurement | Results |
| 1 | 1650 |
| 2 | 1670 |
| 3 | 1620 |

**Table 36. Results of osmolality of Esomeprazole 20mg/15 ml (Example 10) oral solution**

| **Esomeprazole 20mg/15ml solution** | |
|---|---|
| Parameter | Osmolality |
| *Limits* | *1500-2500 mOsm*/*kg* |
| Temperature | 25°C |
| Measurement | Results |
| 1 | 1720 |
| 2 | 1770 |
| 3 | 1500 |

**Conclusion:** Osmolality of omeprazole, pantoprazole and esomeprazole combined oral solutions in mixed form are within the acceptable range 1500 - 2500 mOsm/kg for oral solution. Thus, the combined solution is suitable for direct oral administration.

### Example 12

### Assessment of palatability

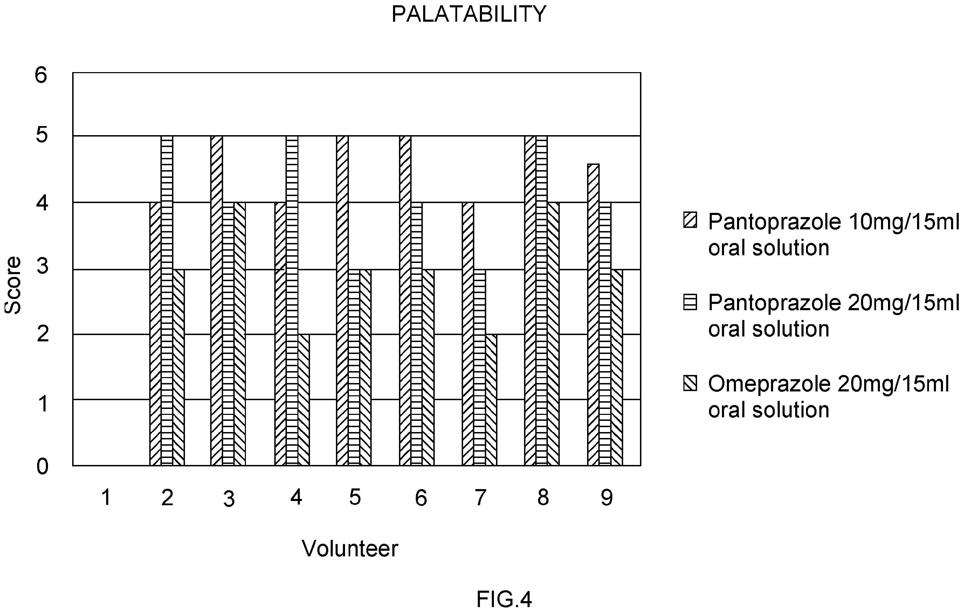

The results of a palatability assessment of each of Examples 6-8 are presented in Table 37 and Fig 4, respectively.

**Table 37. Palatability assessment results**

| **Product** | **Pantoprazole 10mg/15 ml Oral solution** | **Pantoprazole 20mg/15 ml Oral solution** | **Omeprazole 2mg/15ml Oral solution** | **Omeprazole 10mg/15ml Oral solution** |
|---|---|---|---|---|
| *Volunteer* | *Score* | | | |
| 1 | 4 | 5 | 3 | 5 |
| 2 | 5 | 4 | 4 | 5 |
| 3 | 4 | 5 | 2 | 4 |
| 4 | 5 | 3 | 3 | 5 |
| 5 | 5 | 4 | 3 | 5 |
| 6 | 4 | 3 | 2 | 4 |
| 7 | 5 | 5 | 4 | 4 |
| 8 | 4 | 4 | 3 | 5 |

### KEY:

| Taste | **Score** |
|---|---|
| Very good | 5 |
| Good | 4 |
| Moderate | 3 |
| Bad | 2 |
| Very bad | 1 |

The results are shown graphically in Figure 4
From the above data it can be concluded that Pantoprazole 2mg/ml and pantoprazole 4mg/ml showed very good palatability whilst omeprazole 4mg/ml shows a more bitter taste than the others.

The results of palatability assessment of each product are presented in Table 38 and Fig.5, respectively.

**Table 38. Palatability assessment results - esomeprazole vs omeprazole.**

| product | Esomeprazole 10mg/15 ml Oral solution | Omeprazole 1 0mg/15 ml Oral solution | Esomeprazole 20mg/15 ml Oral solution | Omeprazole 20mg/15 ml Oral solution |
|---|---|---|---|---|
| *Volunteer* | *Score* | | | |
| 1 | 5 | 4 | 4 | 3 |
| 2 | 5 | 4 | 4 | 5 |
| 3 | 4 | 5 | 4 | 3 |
| 4 | 3 | 3 | 5 | 3 |
| 5 | 5 | 4 | 4 | 4 |
| 6 | 5 | 3 | 5 | 3 |
| 7 | 4 | 5 | 4 | 4 |
| 8 | 5 | 4 | 5 | 3 |

From the above presented data it can be concluded that Esomeprazole 2mg/ml showed very good palatability results. Esomeprazole 4mg/ml showed very good to good palatability results vs Omeprazole 20mg/15 ml oral solution which showed a moderate palatability score. Specifically, Esomeprazole 20 mg/15 ml oral solution is less bitter than Omeprazole 20 mg/15ml oral solution so there is no need to improve palatability, neither at lower nor for the higher concentration.

## Claims

1. A pharmaceutical formulation comprising:
a first composition being a liquid having a pH of from 11.5 to 12.5 comprising a substituted benzimidazole selected from omeprazole, pantoprazole, and esomeprazole, or an enantiomer, salt, or hydrate thereof, at a concentration between 0.5mg/ml to 5mg/ml,
a phosphate buffering agent,
a base selected from sodium or potassium hydroxide
and
optionally an antioxidant; and
a second composition being a liquid having a pH of from 7 to 9 comprising
a diluent; and
a pH modifying agent which is sodium bicarbonate;
whereby the first and the second compositions are configured to be combined immediately before use to provide a combined liquid medicament having a pH of from 8 to 9.

2. A pharmaceutical formulation according to claim 1 wherein the buffering agent is sodium dihydrogen phosphate.

3. A pharmaceutical formulation according to any preceding claim wherein the antioxidant is present, and is a thiol derivative, preferably N-Acetyl L-Cysteine.

4. A pharmaceutical formulation according to any preceding claim wherein the first and/or second composition comprises one or more additional components selected from the group of dispersing agents, viscosity building agents, sequestering agents, flavouring agents, sweeteners and preservatives.

5. A pharmaceutical formulation according to any preceding claim wherein the first composition has a viscosity of from 150 to 450 centipoise.

6. A pharmaceutical formulation according to any preceding claim wherein the second composition has a viscosity of from 350 to 650 centipoise.

7. A pharmaceutical formulation according to any preceding claim wherein the combined solution has a viscosity of from 300 to 600 centipoise.

8. A pharmaceutical formulation according to any preceding claim wherein the osmolality of the combined solution is from 1500 to 2500 mOsm/kg.

9. A pharmaceutical formulation according to any preceding claim for use as a medicament.

10. A pharmaceutical formulation according to any preceding claim for use in the treatment of a condition selected from gastritis, gastroesophageal reflux disease, dyspepsia, peptic ulcer disease, laryngopharyngeal reflux, gastric and duodenum ulceration and Zollinger-Ellison syndrome.

11. A kit comprising
a first compartment containing a first composition as defined in any one of claims 1 to 8;
a second compartment containing a second composition as defined in any one of claims 1 to 8; and
wherein the first and second compartments are adapted to allow fluid communication between them on actuation to form a combined liquid medicament as defined in any one of claims 1 to 8.

12. A kit as claimed in claim 11 wherein the first and second containers are separated by a breakable membrane.

13. A kit as claimed in claim 12 comprising a threaded cap, wherein turning the cap causes the membrane to break.

## Patentansprüche

1. Pharmazeutische Formulierung, die Folgendes umfasst:
eine erste Zusammensetzung, die eine Flüssigkeit ist, die einen pH von 11,5 bis 12,5 aufweist, die Folgendes umfasst: ein substituiertes Benzimidazol, das aus Omeprazol, Pantoprazol und Esomeprazol ausgewählt ist, oder ein Enantiomer, Salz oder Hydrat davon in einer Konzentration zwischen 0,5 mg/ml bis 5 mg/ml,
ein Phosphat-Puffermittel,
eine Base, die aus Natrium- oder Kaliumhydroxid ausgewählt ist
und
optional ein Antioxidationsmittel; und
eine zweite Zusammensetzung, die eine Flüssigkeit ist, die einen pH von 7 bis 9 aufweist, die Folgendes umfasst:
ein Verdünnungsmittel; und
ein pH-Modifizierungsmittel, das Natriumbicarbonat ist;
wodurch die erste und die zweite Zusammensetzung zur Vereinigung unmittelbar vor der Verwendung konfiguriert sind, um ein vereinigtes flüssiges Medikament bereitzustellen, das einen pH von 8 bis 9 aufweist.

2. Pharmazeutische Formulierung nach Anspruch 1, wobei das Puffermittel Natriumdihydrogenphosphat ist.

3. Pharmazeutische Formulierung nach einem vorstehenden Anspruch, wobei das Antioxidationsmittel vorliegt und ein Thiolderivat, bevorzugt N-Acetyl-L-cystein ist.

4. Pharmazeutische Formulierung nach einem vorstehenden Anspruch, wobei die erste und/oder zweite Zusammensetzung eine oder mehrere zusätzliche Komponenten umfasst, die aus der Gruppe von Folgenden ausgewählt sind: Dispergiermitteln, viskositätsbildenden Mitteln, Maskierungsmitteln, Aromastoffen, Süßstoffen und Konservierungsmitteln.

5. Pharmazeutische Formulierung nach einem vorstehenden Anspruch, wobei die erste Zusammensetzung eine Viskosität von 150 bis 450 mPa.s (Centipoise) aufweist.

6. Pharmazeutische Formulierung nach einem vorstehenden Anspruch, wobei die zweite Zusammensetzung eine Viskosität von 350 bis 650 mPa.s (Centipoise) aufweist.

7. Pharmazeutische Formulierung nach einem vorstehenden Anspruch, wobei die vereinigte Lösung eine Viskosität von 300 bis 600 mPa.s (Centipoise) aufweist.

8. Pharmazeutische Formulierung nach einem vorstehenden Anspruch, wobei die Osmolalität der vereinigten Lösung von 1500 bis 2500 mOsm/kg beträgt.

9. Pharmazeutische Formulierung nach einem vorstehenden Anspruch für die Verwendung als ein Medikament.

10. Pharmazeutische Formulierung nach einem vorstehenden Anspruch für die Verwendung bei der Behandlung eines Leidens, das aus Folgenden ausgewählt ist: Gastritis, gastroösophagealer Refluxkrankheit, Dyspepsie, Magengeschwürerkrankung, laryngopharyngealem Reflux, Magen- und Duodenum-Geschwürbildungen und Zollinger-Ellison-Syndrom.

11. Kit, der Folgendes umfasst:
eine erste Kammer, die eine erste Zusammensetzung, wie in einem der Ansprüche 1 bis 8 definiert, enthält;
eine zweite Kammer, die eine zweite Zusammensetzung, wie in einem der Ansprüche 1 bis 8 definiert, enthält; und
wobei der erste und zweite Kammern geeignet sind, um Flüssigkeitsverbindung zwischen ihnen bei Betätigung zu ermöglichen, um ein vereinigtes flüssiges Medikament, wie in einem der Ansprüche 1 bis 8 definiert, zu bilden.

12. Kit nach Anspruch 11, wobei der erste und zweite Behälter durch eine zerbrechbare Membran getrennt sind.

13. Kit nach Anspruch 12, der eine Gewindekappe umfasst, wobei das Drehen der Kappe das Zerbrechen der Membran bewirkt.

## Revendications

1. Formulation pharmaceutique comprenant :
une première composition qui est un liquide ayant un pH allant de 11,5 à 12,5 comprenant un benzimidazole substitué choisi parmi l'oméprazole, le pantoprazole, et l'ésoméprazole, ou un énantiomère, sel ou hydrate de celui-ci, selon une concentration allant d'entre 0,5 mg/ml à 5 mg/ml,
un agent tampon à base de phosphate,
une base choisie parmi l'hydroxyde de sodium ou de potassium,
et
éventuellement un antioxydant ; et
une deuxième composition qui est un liquide ayant un pH allant de 7 à 9 comprenant
un diluant ; et
un agent de modification du pH qui est le bicarbonate de sodium ;
ce par quoi la première et la deuxième composition sont configurées pour être combinées immédiatement avant utilisation afin de fournir un médicament liquide combiné ayant un pH allant de 8 à 9.

2. Formulation pharmaceutique selon la revendication 1, dans laquelle l'agent tampon est le dihydrogénophosphate de sodium.

3. Formulation pharmaceutique selon l'une quelconque des revendications précédentes, dans laquelle l'antioxydant est présent, et est un dérivé de thiol, préférablement la N-acétyl-L-cystéine.

4. Formulation pharmaceutique selon l'une quelconque des revendications précédentes, dans laquelle la première et/ou la deuxième composition comprend un ou plusieurs composants supplémentaires choisis dans le groupe constitué par les agents de dispersion, les agents de viscosité, les agents séquestrants, les agents aromatisants, les édulcorants et les conservateurs.

5. Formulation pharmaceutique selon l'une quelconque des revendications précédentes, dans laquelle la première composition présente une viscosité allant de 150 à 450 mPa.s (centipoise).

6. Formulation pharmaceutique selon l'une quelconque des revendications précédentes, dans laquelle la deuxième composition présente une viscosité allant de 350 à 650 mPa.s (centipoise).

7. Formulation pharmaceutique selon l'une quelconque des revendications précédentes, dans laquelle la solution combinée présente une viscosité allant de 300 à 600 mPa.s (centipoise).

8. Formulation pharmaceutique selon l'une quelconque des revendications précédentes, dans laquelle l'osmolalité de la solution combinée va de 1500 à 2500 mOsm/kg.

9. Formulation pharmaceutique selon l'une quelconque des revendications précédentes, pour une utilisation comme médicament.

10. Formulation pharmaceutique selon l'une quelconque des revendications précédentes, pour une utilisation dans le traitement d'une condition choisie parmi la gastrite, le reflux gastro-oesophagien, la dyspepsie, l'ulcère gastroduodénal, le reflux laryngo-pharyngé, les ulcères de l'estomac et du duodénum et le syndrome de Zollinger-Ellison.

11. Kit comprenant
un premier compartiment contenant une première composition telle que définie selon l'une quelconque des revendications 1 à 8 ;
un deuxième compartiment contenant une deuxième composition telle que définie selon l'une quelconque des revendications 1 à 8 ; et
dans lequel le premier et le deuxième compartiments sont adaptés pour permettre une communication fluide entre eux lors d'un actionnement pour former un médicament liquide combiné tel que défini selon l'une quelconque des revendications 1 à 8.

12. Kit selon la revendication 11, dans lequel le premier et le deuxième conteneur sont séparés par une membrane rupturable.

13. Kit selon la revendication 12, comprenant un bouchon fileté, où le fait de tourner le bouchon provoque la rupture de la membrane.
